# EUROPEAN PATENT APPLICATION

(11) **EP 4 481 199 A2**
(43) Date of publication of application: **25.12.2024**
(21) Application number: 24211744.8
(22) Date of filing: 22.08.2019
(51) Int. Cl.: F04B 49/00

(54) **82RB ELUTION SYSTEM CONTROL AND CONFIGURATIONS**

(30) Priority: 22.08.2018 US 201862721033 P
(62) Divisional of application: 19852440.7
(71) Applicant: Jubilant Draximage, Inc., Kirkland, Québec H9H 4J4 (CA)
(72) Inventor: RIDDOCH, Robert William, Pierrefonds, QC H9H 1N9 (CA); LEFORT, Etienne, Kirkland, QC H9J 3X6 (CA); DONNELLY, Paul, L'ile Bizard QC H9E 1R3 (CA); SANTOPIETRO, Riccardo, Pierrefonds, QC H9K 1R7 (CA); MOORE, Thomas Allen, Beaconsfield, QC H9W 4Z7 (CA)
(74) Representative: Herrero & Asociados, S.L.

(57) **Abstract**

Disclosed are new configurations of a rubidium elution system that offer several advantages. The rubidium elution systems of the present disclosure comprise an assembly including a ⁸²Sr/⁸²Rb generator, an activity detector, a waste container, a pump, a sensor, a tubing circuit and at least one valve. The waste container is located in the lower section of the assembly. More particularly, the top surface of the waste container compartment is either below the top surface of the generator compartment, or the waste container is at the same elevation than the generator or below the pump.

## Description

### TECHNICAL FIELD

The present disclosure relates generally to nuclear medicine and specifically concerns ⁸²Sr/⁸²Rb elution systems.

### BACKGROUND OF THE INVENTION

The subject matter disclosed herein relate to radiopharmaceutical substances with short half-life, such as Rubidium (⁸²Rb). Rubidium (⁸²Rb) is used as a positron emission tomography (PET) tracer for non-invasive determination of myocardial perfusion (blood flow).

Typical ⁸²Sr/⁸²Rb elution systems comprise a reservoir of sterile saline solution (e.g., 0.9% Sodium Chloride Injection), one or more pumps to pump the sterile saline solution from reservoir, a source for generating radioactive isotopes in solution, a radioactivity detector to measure activity of different isotopes, a dose calibrator, a waste container, set of infusion tubing assembly, one or more sensors, a computer, and shielded enclosures on a platform meant to move the elution system. During operation of such systems, the pump moves the saline solution from the reservoir and through the generator to elute the ⁸²Rb which is eluted in the form of ⁸² RbCl and also to regulate the flow of sterile saline solution to the bypass line. The radioactive solution which exits the generator is then infused to a patient to be diagnosed via a patient outlet. As known among those skilled in the art, ⁸²Rb is generated by radioactive decay of the ⁸²Sr, and thus the rate of production of ⁸²Rb at any given time, is a function of the activity of ⁸²Sr remaining in the generator. The rate of ⁸²Rb production exponentially decreases through the useful life of the generator, directly in correlation with the half-life of ⁸²Sr.

The various shielded and non-shielded components in the elution system play a role in providing a desired dose, and ensuring a safe and an easy to clean environment. All known ⁸²Sr/⁸²Rb generator systems like Cardiogen-82^{®} and RUBY-FILL^{®} have a waste container that is on top of their respective structures, and at a higher elevation relative to the generator and/or the pump. The present inventors have identified alternate configurations of elution system that offer several advantages.

### SUMMARY OF THE INVENTION

An object of the present disclosure is to provide new configurations of a rubidium elution system wherein the waste container is located in a lower section of an assembly that contains the various components of the system, such as at a lower elevation than the pump, at the same elevation than the generator, or at an elevation where the top surface of the compartment housing the waste container is lower than the elevation of the top surface of the compartment housing the generator. Another object of the disclosure is to provide safety means that are adapted to these new configurations of a rubidium elution system.

Another object of the present disclosure is to provide a method for manufacturing said new configurations of rubidium elution system.

The present disclosure concerns any of the following items:
Item 1. A rubidium elution system comprising an assembly that includes a ⁸²Sr/⁸²Rb generator, an activity detector, a waste container, a pump, a sensor, a tubing circuit, and at least one valve; wherein:
   the assembly has a lower section and a upper section, and
   the waste container is located in the lower section of the assembly.
Item 2. The rubidium elution system of item 1, wherein the lower section consists of the lower two third of the assembly.
Item 3. The rubidium elution system of item 2, wherein the lower section consists of the lower half of the assembly.
Item 4. The rubidium elution system of any one of item 1, 2 or 3, wherein:
   the waste container is enclosed in a waste compartment that has a top surface, the top surface of the waste compartment is at a first elevation within the assembly;
   the generator is enclosed in a generator compartment that has a top surface, the top surface of the generator compartment is at a second elevation within the assembly; and
   the first elevation is lower than the second elevation.
Item 5. The rubidium elution system of any one of items 1, 2 or 3, wherein:
   the waste container is enclosed in a waste compartment;
   the generator is enclosed in a generator compartment; and
   the waste compartment is at an elevation within the assembly that is the same as an elevation at which the generator compartment is located.
Item 6. The rubidium elution system of any one of items 1 to 5, wherein the waste container is at an elevation that is lower than the pump.
Item 7. The rubidium elution system of item 4 or 5, wherein the waste container is housed within an enclosure that may be removed from the waste compartment.
Item 8. The rubidium elution system of item 4 or 5, wherein the waste compartment includes a door for accessing the waste container.
Item 9. The rubidium elution system of item 4 or 5, including a sliding mechanism or a lifting mechanism for removing the waste container from the waste compartment.
Item 10. The rubidium elution system of any one of items 1 to 9, wherein the assembly further comprises a dose calibrator that is configured to detect at least one radioisotope.
Item 11. The rubidium elution system of item 10, further comprising locking means for preventing a user of the system from changing a configuration of the dose calibrator.
Item 12. The rubidium elution system of item 11, further comprising an interface for configuring the dose calibrator, and wherein the locking means prevents the user from changing a configuration of the dose calibrator by locking the interface.
Item 13. The rubidium elution system of item 12, wherein the locking means comprises a compartment that encloses the interface, and the compartment is locked in order to prevent the user from accessing the interface.
Item 14. The rubidium elution system of any one of items 1 to 13, wherein the assembly further comprises a computer.
Item 15. The rubidium elution system of any one of items 1 to 14, wherein the assembly is positioned on a platform.
Item 16. The rubidium elution system of item 15, wherein the platform is mobile.
Item 17. The rubidium elution system of item 16, wherein the platform has wheels and a mechanism for braking and locking the wheels.
Item 18. The rubidium elution system of any one of items 1, 2 or 3, wherein the ⁸²Sr/⁸²Rb generator and the waste container are each enclosed in respective shielded compartments.
Item 19. The rubidium elution system of any one of items 4 or 5, wherein the waste compartment and the generator compartment are shielded.
Item 20. The rubidium elution system of any one of items 1 to 19, wherein the assembly is housed in a cabinet structure.
Item 21. The rubidium elution system of any one of items 1 to 20, further comprising a controller that is configured to control the pump and the at least one valve that permit delivery of a radioactive solution at a constant radioactivity rate.
Item 22. The rubidium elution system of any one of items 1 to 21, wherein the pump is a syringe pump or a peristaltic pump.
Item 23. The rubidium elution system of item 22, wherein the pump is a peristaltic pump.
Item 24. The rubidium elution system of item 23, wherein the peristaltic pump comprises a motor a pump component, and the peristaltic pump is horizontally oriented such that the motor is located at an elevation that is the same as that of the pump component.
Item 25. The rubidium elution system of any one of items 1 to 24, wherein said at least one valve comprises a valve upstream within the system relative to the generator and a valve downstream from the generator.
Item 26. The rubidium elution system of item 25, further comprising a bypass line that connects the valve upstream relative to the generator and the valve from downstream the generator.
Item 27. The rubidium elution system of item 25 or 26, wherein the at least one valve includes a pinch valve or a divergence valve.
Item 28. The rubidium elution system of item 27, wherein the at least one valve includes a pinch valve.

Other objects, advantages and features of the present disclosure will become more apparent upon reading of the following non-restrictive description of specific embodiments thereof, given by way of examples only with reference to the accompanying drawings.

### BRIEF DESCRIPTION OF DRAWINGS

**FIG. 1** shows a perspective view of an embodiment of a rubidium elution system according to the present disclosure in which the front door of the cabinet structure is opened and provides access to the compartment housing the waste container and the compartment housing the generator; and where the door of compartment housing the waste container is also opened.
**FIG. 2** provides a close-up view of an embodiment of the compartment housing the waste container where the door of the compartment is opened and the waste container is housed within an enclosure that may be removed from its compartment.
**FIG. 3** is a front view of an embodiment of a rubidium elution system in which the front door and the top panel of the cabinet structure are opened, the door of compartment housing the waste container is also opened, and the wheels under the cabinet structure and a screen installed above the cabinet structure are shown.
**FIG. 4** shows a peristaltic pump according to an embodiment of the present disclosure that is horizontally oriented such that the motor and the pump component of the pump system are at the same elevation.
**FIG. 5** illustrates the tubing circuit of an embodiment of the present disclosure, comprising an eluant line, a bypass line, a waste line and a patient line.

### DETAILED DESCRIPTION

As used herein, the terms "elution system", "rubidium elution system" and "⁸²Sr/⁸²Rb elution system" can be used interchangeably and refer to a strontium-rubidium infusion system for use in generating a radioactive solution containing rubidium-82, measuring the radioactivity in the solution generated by the system, and infusing the radioactive solution to a patient in order to perform various studies on a patient's organ, such as heart or kidney.

As used herein, the terms "cart" or "system" or "trolley" are intended to encompass a platform. Said platform can be mobile or stationary.

The material used for "shielding" is made up of any radiation attenuating material, including, but not limited to, depleted uranium (U), lead (Pb), tin (Sn), antimony (Sb), tungsten (W), bismuth (Bi), any other material, or any combination thereof, as long as it provides a barrier to the radioactivity of rubidium or strontium

As used herein, the term "shielded component" refers to components of the system that are surrounded by a shielding material, and may refer to the generator, the dose calibrator, the activity detector, the waste container, and/or the tubing line or a part of the tubing line.

As used herein, the term "non-shielded components" refers to components of the system that are not shielded, such as the pump, the valves, the saline reservoir, the computer, the controller of the system, the interface of the dose calibrator, and/or the tubing line or a part of the tubing line.

As used herein, the term "cabinet structure" refers to an outer structure of the system that extend upward from a platform and houses an assembly. The cabinet structure may be formed of any radiation resistant material, including, but not limited to, stainless steel, injection-molded polyurethane, or any other suitable materials or combination thereof.

As used herein, the term "supporting accessories" refers to items such as wheels, lever, paddle, additional saline reservoir, drawer system for housing vials, and a handle for facilitating movement of the system.

As used herein, the expression "locking means for preventing access to the interface by a user of the system" refers to any mechanism, system, or technology to provide security against access by a user to the interface, such as a locked compartment enclosing the interface of the dose calibrator, the requirement for input of a security code or security data (RFID, biometric, numeric, audible, or the like) for modifying the parameters of the interface, integrating the interface into the dose calibrator in such a way that the interface is rendered not accessible, or the like. In an embodiment, only the manufacturer have access to the interface of the dose calibrator. In another embodiment, no one has access to the interface. Being placed outside the primary assembly, the dose calibrator is therefore accessible and it is advantageous to prevent a user from modifying the interface parameters in order to ensure that the dose calibrator remains set properly for the rubidium elution system of the present disclosure.

The generator column may be prepared in accordance with U.S. Pat. No. 8,071,959, the entire contents of which are incorporated herein by reference.

The generator system should be able to perform all the desired functions without unwanted and hazardous effects. ⁸²Rb has a half-life of 75 seconds, and its potential impurities, ⁸²Sr and ⁸⁵Sr, have a half-life of 25.3 days and 64.8 days, respectively. It is essential that patients are not exposed to these undesired isotopes, of which both are characterized by a long half-life. Accordingly, in order to ensure adequate safety, quality control testing of the generator is performed on daily basis prior to use. The daily quality control process for the generator includes collecting an eluted sample in a shielded vial for evaluation, and measuring the activity of the sample in a dose calibrator. Further, the sample is retained to permit decay of all active rubidium, after which a second stage of breakthrough testing is performed. The measured initial activity and the final activity are used to calculate the breakthrough information. The United States Pharmacopeia (USP) provides relevant regulatory limits for breakthrough of strontium of 0.02 µCi of ⁸²Sr/mCi of ⁸²Rb activity and 0.2 µCi of ⁸⁵Sr/mCi of ⁸²Rb activity. Further, to ensure a higher degree of safety, a system may impose a more stringent limit of 50% of the USP limits.

A constant activity-rate refers to an activity-rate delivered with respect to time wherein the activity-rate is constant with respect to a predetermined constant activity concentration. A method to deliver a constant activity-rate dose is described in U.S. Pat. No. 7,813,841, which is incorporated herein by reference.

Different elution strategies that can be used for patient infusions include:
Constant-Activity elutions, which allow the user to enter the desired activity and duration of the elution. The system automatically estimates a flow rate and controls flow through the generator or a bypass line by a comparison algorithm to achieve activity around the desired set point.

Constant-Flow elutions, which allow the user to specify the desired activity and the flow rate. The elution duration is automatically estimated based on the activity vs. volume curve measured during calibration.

Constant-Time elutions, which allow the user to specify the desired activity and time for the elution. The flow rate is automatically calculated based on the activity vs. volume curve measured during calibration.

In certain embodiments, the rubidium elution system includes a controller configured to control the pump and the at least one valve so as to deliver a solution of rubidium at a constant activity rate to a patient.

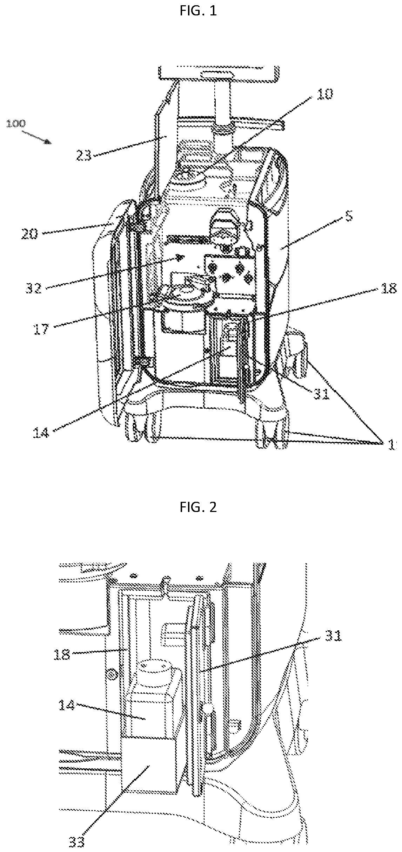

FIG. 1 is a front view of a rubidium elution system 100 of the present disclosure where the assembly 32 is enclosed into a cabinet structure 5. The cabinet structure 5 has a front door 20 that gives access to the waste compartment 18 housing the waste container 14 and access to the generator compartment 17 housing the generator 7 (not shown) that is housed in a generator compartment 17. In the embodiment shown in FIG. 1, the waste compartment 17 has a waste door 31 that provides access to the waste container 14. Preferably, the waste container 14 is housed within an enclosure 33 that may be removed from its shielded compartment 18 when the waste door 31 is opened, as shown in FIG. 2. In a certain embodiment, the waste container 14 can be accessed from the waste container compartment 18 horizontally by mounting a waste platform on rails. In this embodiment, the waste platform is slid out horizontally prior to pulling out the waste container 14 from its compartment (sliding out mechanism). In another compartment, the waste container 14 is removed by a lifting mechanism that can be automated. In the embodiment of FIG. 1, the dose calibrator 10 can be accessed by opening a top panel 23 of the cabinet structure 5.

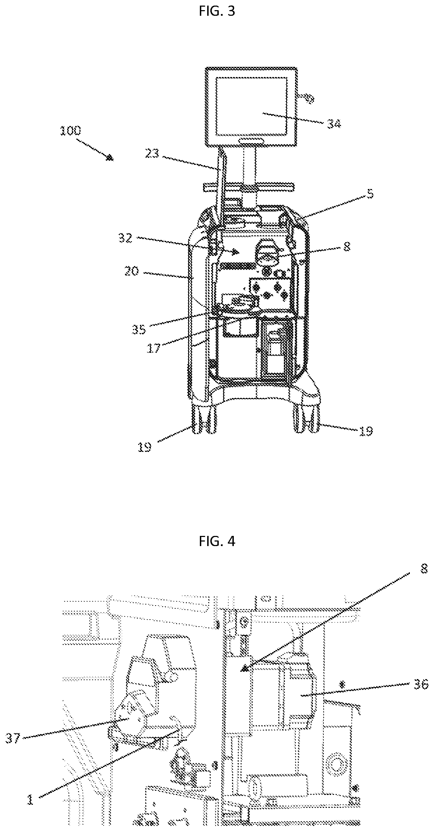

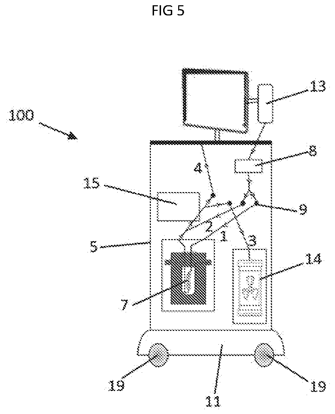

FIG. 3 provides a front view of an embodiment of the rubidium elution system 100 according to the present disclosure, where the assembly 32 is positioned on a platform 11 (shown in FIG. 5) that is mobile. In this embodiment, mobility is provided by means of wheels 19, and it can be manually or motor driven. As it can be seen in FIGS. 1 and 3, the assembly 32 and the platform 11 are preferably recovered by a cabinet structure 5. The platform 11 preferably includes two pairs of wheels 19 mounted on a bottom side of the platform, for allowing the mobility of the elution system 100. In some embodiments, the platform 11 includes a mechanism for braking and locking the wheels 19, such as a lever.

In an embodiment as the one shown in FIG. 3, the rubidium elution system further comprises a computer screen 34 for displaying the information about the rubidium elution and the patient infusion. Preferably, the screen 34 is a touch screen where the user may provide inputs to the computer (not shown) about patient information and on the desired dose of rubidium. The computer is preferably integrated into the assembly 32.

Preferably, the waste door 31 of the waste compartment 18 has an upper edge and lower edge, the upper edge of the waste door 31 is located at a lower elevation than the elevation of the opening 35 of the generator compartment 17. In an embodiment, the elevation of the upper edge of the waste door 31 is from about 5 inches to about 12 inches above the platform 11, more preferably from about 10 to about 12 inches above the platform 11, and further preferably at 10±0.5 inches above the platform 11, or further preferably at 11±0.5 inches above the platform 11, or further preferably at 12±0.5 inches above the platform 11. In another embodiment, the lower edge of the waste door 31 is from about 15 inches to about 30 inches above the platform 11. In certain aspects, the assembly 32 is defined with an upper section and a lower section. According to the present disclosure, the waste container 14 is preferably located in the lower section of the assembly 32. In an embodiment, the lower section represents the lower two third of the assembly 32. In another embodiment, the lower section represents the lower half of the assembly 32. Positioning the waste container 14 in the lower section of the assembly 32 offers several advantages for the user and for the system, such as distancing the waste container from the user's eyes, lowering the center of gravity that provides higher stability of the system, making it easier to clean and decontaminate in case of a spilling or overflow, and easier to replace the waste container, and preventing any waste overflow to run through electronic components that may result in shorting-out the system. It also leaves additional space for the pump 8 that is usually located on the upper portion of the assembly 32. More particularly, the new configurations allow the pump 8 to be horizontally oriented, as illustrated in FIG. 4. Advantageously, horizontal orientation is the typical orientation of the peristaltic pump, and is recommended by the manufacturer.

In certain aspects, the term "about" preferably refers to 20% of the corresponding value. In other aspects, the term "about" preferably refers to 10% of the corresponding value.

According to certain preferred embodiments, the top surface of the waste compartment 18 is at a first elevation; and the top surface of the generator compartment 17 is at a second elevation; and the first elevation is lower than the second elevation.

According to another preferred embodiment, the waste compartment 18 and the generator compartment 17 are at the same elevation.

According to a further preferred embodiment, the waste container 14 is at an elevation, which is lower than the pump 8.

In the presently disclosed rubidium elution systems, when emptying the waste container 14 is necessary, the user may i) open the door 20 of the cabinet structure, ii) open the door 31 of the waste compartment 18, iii) pull horizontally the enclosure 33, iv) disconnect the waste line (not shown) attached thereto, and v) lift the waste container 14 so as to remove it from its enclosure 33.

In certain embodiments, the ⁸²Sr/⁸²Rb generator, the dose calibrator, the waste container, and the activity detector are enclosed within respective shielded compartments. Preferably, each of the ⁸²Sr/⁸²Rb generator, the dose calibrator, the waste container, and the activity detector is enclosed within a respective shielded compartments. The door 31 of the waste compartment 18 is preferably shielded. The opening of the generator is also preferably shielded.

FIG. 5 shows a front view of a rubidium elution system according to the present disclosure. The tubing circuit includes an eluant line 1 that provides an eluant (preferably a saline solution contained in a saline reservoir 13) to the generator 7 by means of a pump 8. The tubing circuit also includes a bypass line 2 connected to the eluant line 1 that carries the eluant to the tubing circuit downstream the generator 7 such that the eluant bypasses the generator 7. The tubing circuit further includes a waste line 3 that directs into a waste container 14 a mixture composed of the eluant fed by the bypass line 2 and the radioactive eluate that has exited the generator 7. The tubing circuit also further includes a patient line 4 that directs the same mixture to either i) a patient for infusing said patient with a rubidium solution, or ii) a vial located in the dose calibrator 10 for system's calibration and strontium breakthrough test. The activity detector 15 can be a beta detector, a positron detector, or a beta and positron detector. In certain embodiments, the rubidium elution system 100 comprises at least one valve 9. Preferably, said at least one valve 9 comprises a valve upstream the generator 7 and a valve downstream the generator 7. Said valves 9 can be embodied by pinch valves and/or divergence valves.

In certain embodiments, the pump 8 that can be embodied by a syringe pump, a peristaltic pump, or another type of pump. In the embodiment of the present invention shown in FIG. 4, the pump 8 is a peristaltic pump and is mounted horizontally on the front panel of the assembly 32. The expression "horizontally mounted" or "horizontally oriented" means that the motor and the pump component of the pump assembly 8 are at the same elevation. The pump 8 is preferably located from about 28 inches to about 36 inches above the platform 11.

The present disclosure includes embodiments where the dose calibrator is accompanied with its own interface that configures the dose calibrator for detection of rubidium, and with embodiments where the calibrator has no interface and is controlled by the computer. In a preferred embodiment, the system further includes a locking means for preventing a user from reconfiguring the dose calibrator in such a way that the dose calibrator remains configured as initially configured by the manufacturer. In a preferred embodiment, the dose calibrator is configured to detect rubidium and/or strontium. In a further preferred embodiment, the dose calibrator is configured to detect rubidium. Since strontium and rubidium are in an instant equilibrium, one can use the measured quantity of rubidium to calculate the quantity of strontium that is present in a solution after the initial content in rubidium has decayed. On way to prevent a user from reconfiguring the dose calibrator is to prevent access to the interface of the dose calibrator. In an embodiment, the locking means comprises an identification system for allowing access to the interface and reconfiguring the dose calibrator by the manufacturer of the system and not the user of the system. In another embodiment, the locking means comprises a locked compartment 30 as shown in FIGS. 5 and 7, which encloses the interface of the dose calibrator 10 and prevents access to by the user of the system. Preferably, the manufacturer of the system remains able to unlock the compartment of the interface and able to configure or reconfigure the dose calibrator as needed. In a variant of this embodiment, the locked compartment enclosing the interface of the dose calibrator 10 is provided by a second cabinet structure 6 that also encloses or partly encloses the dose calibrator 10. The compartment enclosing the interface can be included in the cabinet structure. In another embodiment the interface is integrated in the dose calibrator and not accessible to the user. In a further embodiment, there is no interface, and the computer does not allow the user to modify the configurations of the dose calibrator.

In certain aspects, the computer of the rubidium elution system is configured to proceed with a quality control test (breakthrough tests) at pre-determined time, upon user's request and at least once a day. The computer is configured to prevent a patient infusion when the quality control test result determines that the strontium concentration is equal to or above 0.01 µCi of ⁸²Sr/mCi of ⁸²Rb activity or equal to or above 0.1 µCi of ⁸⁵Sr/mCi of ⁸²Rb activity.

In other aspects, the rubidium elution system comprises a computer screen includes a speaker for providing an audible or visible alert for one or more different operations of system.

In certain embodiments, the rubidium elution system comprises a computer having a processor and a memory communicatively connected to the processor when the system is functioning. Memory has processor-executable instructions that, when executed on the processor, cause the system to perform representative functions of the system. Certain embodiments may also be provided as a computer-implemented method. Additionally, certain embodiments may be implemented as computer-executable instructions stored on computer-readable storage media. Computer readable storage media may be distinguished from computer-readable communications media that include transitory signals.

While the presently disclosed subject matter has been described in detail with reference to certain preferred embodiments, it should be appreciated that the present disclosure is not limited to those precise embodiments. Rather, in view of the present disclosure, which describes the current best mode for practicing the disclosed embodiments, many modifications and variations would present themselves to those skilled in the art without departing from the scope, and spirit of the presently disclosed subject matter.

## Claims

1. An ⁸²Sr/⁸²Rb elution system (100) having a cabinet structure (5), comprising: shielded components, and non-shielded components, wherein:
a) the shielded components comprises: a generator compartment (17), a waste compartment (18), a dose calibrator (10), and an activity detector (15), and
b) the non-shielded components comprises: a pump (8), valves (9), a saline reservoir (13), a computer, a controller of the system, an interface of the dose calibrator (10), tubing line (1, 2, 3, 4),
wherein the waste compartment (18) has a top surface, and a door (31) for accessing the waste container (14), and the generator compartment (17) has a top surface,
wherein the top surface of the waste compartment (18) is at a first elevation; and the top surface of the generator compartment (17) is at a second elevation; and the first elevation is lower than the second elevation, and
wherein the waste compartment (18) includes a sliding mechanism for removing the waste container (14) from the waste compartment (18) horizontally.

2. An ⁸²Sr/⁸²Rb elution system (100) having an assembly (32), enclosed into a mobile cabinet structure (5), comprising: shielded components, and non-shielded components; wherein:
a) the shielded components comprises: a generator compartment (17), a waste compartment (18), a dose calibrator (10), and an activity detector (15), and
b) the non-shielded components comprises: a pump (8), valves (9), a saline reservoir (13), a computer, a controller of the system, an interface of the dose calibrator (10), tubing line (1, 2, 3, 4),
wherein the waste compartment (18) has a door (31) for accessing the waste container (14), and the waste container (14) is housed within an enclosure (33) that is removed horizontally from the waste compartment (18) through a sliding mechanism when the waste door (31) is opened, wherein the waste compartment (18) and the generator compartment (17) have top surfaces, and wherein the top surface of the waste compartment (18) is at a first elevation; and the top surface of the generator compartment (17) is at a second elevation; and the first elevation is lower than the second elevation.

3. The ⁸²Sr/⁸²Rb elution system of claim 1, wherein the cabinet structure is housed in a mobile platform (11).

4. The ⁸²Sr/⁸²Rb elution system of claim 1, wherein the controller is configured to control the pump (8) and the at least one valve (9) that permit delivery of a radioactive solution.

5. The ⁸²Sr/⁸²Rb elution system of claim 1, wherein the cabinet structure (5) further comprises front door (20) and top panel (23), wherein the front door (20) and the top panel (23) of the cabinet structure (5) are opened and provides access to the waste compartment (18) housing of the waste container (14) and the generator compartment (17) housing of the generator (7).

6. The ⁸²Sr/⁸²Rb elution system of claim 1, wherein the elution system further comprises a computer screen (34).

7. The ⁸²Sr/⁸²Rb elution system of claim 6, wherein the computer screen (34) is a touch screen that provides inputs to the computer about patient information and on the desired dose of rubidium.

8. The ⁸²Sr/⁸²Rb elution system of claim 1, wherein the door (31) of the waste compartment (18) has an upper edge and lower edge.

9. The ⁸²Sr/⁸²Rb elution system of claim 8, wherein the upper edge of the waste door (31) is located at a lower elevation than the elevation of the opening (35) of the generator compartment (17).

10. The ⁸²Sr/⁸²Rb elution system of claim 1, wherein the dose calibrator (10) further comprises locking means for preventing a user of the system (100) from changing a configuration of the dose calibrator (10).

11. The ⁸²Sr/⁸²Rb elution system of claim 1, wherein the at least one valve includes a pinch valve or a divergence valve.

12. An ⁸²Sr/⁸²Rb elution system (100) having an assembly (32), enclosed into a mobile cabinet structure (5), comprising: shielded components, and non-shielded components, wherein:
a) the shielded components comprises: a generator compartment (17), a waste compartment (18), a dose calibrator (10), and an activity detector (15), and
b) the non-shielded components comprises: a pump (8), valves (9), a saline reservoir (13), a computer, a controller of the system, an interface of the dose calibrator (10), tubing line (1, 2, 3, 4),
wherein the waste compartment (18) and the generator compartment (17) have top surfaces, wherein the top surface of the waste compartment (18) is at a first elevation; and the top surface of the generator compartment (17) is at a second elevation; and the first elevation is lower than the second elevation, and
wherein the waste compartment (18) has a door (31) for accessing the waste container (14) within an enclosure (33) that is removable horizontally from the waste compartment (18) through a sliding mechanism by mounting a waste platform on rails when the waste door (31) is opened.

13. The ⁸²Sr/⁸²Rb elution system of claim 12, wherein:
the tubing line includes: an eluant line (1) that provides an eluant or a saline solution contained in a saline reservoir (13) to the generator (7) by means of a pump (8);
a bypass line (2) connected to the eluant line (1) that carries the eluant to the tubing circuit downstream the generator (7) such that the eluant bypasses the generator (7);
a waste line (3) that directs into a waste container (14); and
a patient line (4) that directs the same mixture to either i) a patient for infusing a radioactive eluate solution, or ii) a vial located in the dose calibrator (10) for calibration and strontium breakthrough testing.

14. The ⁸²Sr/⁸²Rb elution system of claim 12, further comprises a supporting accessories wherein the supporting accessories can be wheels, lever, paddle, additional saline reservoir, drawer system for housing vials, and a handle for facilitating movement of the system.

15. The ⁸²Sr/⁸²Rb elution system of claim 1, wherein the shielded component comprises:
a) a waste compartment (18), wherein the waste compartment (18) is at a lower elevation than the generator compartment (17) and the pump (8),
b) a generator compartment (17), wherein the generator compartment (17) is at a higher elevation than the waste compartment (18), and
c) a dose calibrator (10), wherein the dose calibrator (10) is housed at the top surface of the mobile cabinet structure (5) than the waste compartment (18) and the generator compartment (17).
